# EUROPÄISCHE PATENTSCHRIFT

(11) **EP 1 280 840 B1**
(45) Veröffentlichungstag und Bekanntmachung des Hinweises auf die Patenterteilung: **04.10.2006**
(21) Anmeldenummer: 01947263.8
(22) Anmeldetag: 07.05.2001
(51) Int. Cl.: C08G 18/28, C08G 18/48, C08G 18/70, C08G 18/61, A61K 8/87

(54) **POLYURETHAN UND DESSEN VERWENDUNG ZUR MODIFIZIERUNG RHEOLOGISCHER EINGENSCHAFTEN**
POLYURETHANE AND THE USE THEREOF FOR MODIFYING RHEOLOGICAL PROPERTIES
POLYURETHANNE ET SON UTILISATION POUR LA MODIFICATION DE PROPRIETES RHEOLOGIQUES

(30) Priorität: 08.05.2000 DE 10022247
(43) Veröffentlichungstag der Anmeldung: 05.02.2003
(73) Patentinhaber: BASF Aktiengesellschaft, 67056 Ludwigshafen (DE)
(72) Erfinder: MEFFERT, Helmut, 68167 Mannheim (DE); NGUYEN KIM, Son, 69502 Hemsbach (DE)
(74) Vertreter: Kinzebach, Werner
(86) Internationale Anmeldenummer: PCT/EP2001/005163
(87) Internationale Veröffentlichungsnummer: WO 2001/085821

(56) Entgegenhaltungen:
- EP-A- 0 307 775
- EP-A- 0 601 383
- EP-A- 0 875 557
- EP-A- 0 913 414
- WO-A-97/31046
- US-A- 4 079 028
- US-A- 5 281 654

## Beschreibung

Die vorliegende Erfindung betrifft ein Polyurethan, dessen Verwendung zur Modifizierung der rheologischen Eigenschaften von homogenphasigen oder heterogenphasigen Zusammensetzungen, die wenigstens eine hydrophobe Verbindung enthalten, sowie Zusammensetzungen, die diese Polyurethane enthalten.

Zur Herstellung von kosmetischen, pharmazeutischen und technischen Produkten werden häufig hydrophobe Substanzen eingesetzt, die bei Raumtemperatur flüssig sind. Bei diesen Flüssigkeiten handelt es sich z. B. um aliphatische oder cycloaliphatische Kohlenwasserstoffe und Kohlenwasserstoffgemische, Öle, Fette und andere kommerziell erhältliche Lösungsmittel, die gar nicht oder nur zu einem geringen Teil in Wasser löslich sind. An kosmetische, pharmazeutische und technische Mittel werden häufig spezielle Anforderungen bezüglich ihrer rheologischen Eigenschaften gestellt. Häufig können sie nur mit Hilfe von Zusatzstoffen, sogenannten Verdickern, in die gewünschte Anwendungsform gebracht werden. Beispiele für übliche niedermolekulare Verdickungsmittel sind z. B. die Alkali- und Aluminium-Salze von Fettsäuren, Fettalkohole oder Wachse. Die Verwendung der bekannten Verdickungsmittel ist jedoch, je nach Einsatzgebiet der zu verdickenden Zubereitung, häufig mit Nachteilen verbunden. So kann entweder die Verdickungswirkung der Verdickungsmittel nicht zufriedenstellend, ihr Einsatz unerwünscht oder ihre Einarbeitung in die zu verdickende Zubereitung beispielsweise wegen ihrer Unverträglichkeit mit der zu verdickenden wasserunlöslichen Verbindung erschwert oder ganz unmöglich sein. Ein aktueller Anspruch, der speziell an kosmetische und pharmazeutische Mittel gestellt wird, ist, den Anteil an nicht wirkaktiven Substanzen, wie Verdickern, möglichst gering zu halten.

Es ist bekannt, zur Modifizierung der rheologischen Eigenschaften von im Wesentlichen wasserunlöslichen Flüssigkeiten Polymere einzusetzen. Diese haben den Vorteil, dass sie, sofern sie in der hydrophoben Substanz löslich sind, im Allgemeinen eine Einstellung der Viskosität in Abhängigkeit von ihrem Molekulargewicht ermöglichen.

Ein häufig auftretender Nachteil beim Einsatz von Polymeren als Verdickungsmittel zur Herstellung von höherviskosen bzw. wachs- oder gelartigen Zubereitungen ist, dass mit zunehmendem Molekulargewicht des Polymers dessen Einarbeitung im Allgemeinen schwerer wird, und dass schließlich oftmals nur noch eine Quellung des Polymers anstatt der angestrebten Lösung zu beobachten ist. Werden zur Herstellung derartiger Formulierungen Lösungsmittel als Hilfsmittel eingesetzt, so lassen sich diese oftmals nicht mehr vollständig aus der Mischung entfernen.

Die WO-A-98/17243 und die WO-A-98/17705 beschreiben den Einsatz esterterminierter Polyamide zur Formulierung transparenter Gele aus flüssigen Kohlenwasserstoffen mit geringer Polarität. Nachteilig am Einsatz dieser Polyamide ist, dass die resultierenden Gele mit der Zeit vergilben und sich verschiedene vollsynthetische Öle, wie Siliconöle, auf diese Weise nicht verdicken lassen.

Die WO-A-97/36572 beschreibt eine Basiszusammensetzung zur Herstellung kosmetischer Mittel, die wenigstens ein flüssiges Silicon und wenigstens ein gelbildendes Mittel enthält. Als Gelbildner werden Polymere eingesetzt, die Siloxangruppen und polare, zur Ausbildung von Wasserstoffbrückenbindungen geeignete Gruppen eingebaut enthalten. Letztere sind ausgewählt unter Estergruppen, Urethangruppen, Harnstoffgruppen, Thioharnstoffgruppen und Amidgruppen. Zwar wird in diesem Dokument ganz pauschal darauf hingewiesen, dass die Gelbildner auch z. B. durch C₁- bis C₂₀-Monoalkohole endständig abgesättigt sein können. Der Einsatz von Polyurethanen, die wenigstens ein Diisocyanat, wenigstens einen Polyether und wenigstens eine Verbindung mit einem aktiven Wasserstoffatom und einer langkettigen Alkyl- oder Alkenylgruppe eingebaut enthalten, als Gelbildner, wird jedoch nicht beschrieben. Zudem sind die beschriebenen Polymere bezüglich ihrer Verdikkungswirkung noch verbesserungswürdig. So lassen sich kosmetische Formulierungen in Stiftform nur bei Einsatz sehr großer Mengen an Verdicker, im Bereich von etwa 60 Gew.-%, bezogen auf die Gesamtformulierung, oder mit Hilfe zusätzlicher Emollientien (Wachsen), wie z. B. C₁₂- bis C₁₅-Alkyllactaten, erzielen. Bei der Formulierung vom Mehrphasensystemen müssen zwingend Tenside verwendet werden. Zudem eignen sich die als Gelbildner eingesetzten Polymere nicht als Verdicker für Öle, die nicht auf Silicon basieren. Auch die Verdickung von Ölgemischen aus Siliconölen und davon verschiedenen weiteren Ölen wird in dieser Schrift nicht beschrieben.

Die WO-A-97/36573 beschreibt eine Basiszusammensetzung zur Herstellung kosmetischer Mittel, die wenigstens ein flüssiges Silicon und einen Gelbildner auf Basis einer Kombination eines Wachses und eines Polyamids enthält. Dabei ist das Wachs und/oder das Polymer mit einer Siloxangruppe modifiziert. Die als Gelbildner eingesetzte Mischung zielt speziell auf die Herstellung von Formulierungen mit einem hohen Siliconölanteil. Eine Verdickung von Gemischen aus Siliconölen und anderen Ölen bzw. ausschließlich von nicht auf Silicon basierenden Ölen wird nicht beschrieben.

Die unveröffentlichte deutsche Patentanmeldung P 199 41 365.7 beschreibt Urethan- und/oder Harnstoffgruppen-haltige Oligomere und deren Verwendung zur Modifizierung der rheologischen Eigenschaften von hydrophoben Flüssigkeiten. Der Einsatz von Polyurethanen, die wenigstens eine Verbindung mit einem aktiven Wasserstoffatom und einer längerkettigen Alkyl- oder Alkenylgruppe eingebaut enthalten, als verdicker, wird in diesem Dokument nicht beschrieben.

Der vorliegenden Erfindung liegt die Aufgabe zugrunde, eine Zusammensetzung zur Verfügung zu stellen, die 20 bis 99.9 Gew.-% wenigstens einer hydrophoben, bei Umgebungstemperatur flüssigen Verbindung enthält und deren rheologische Eigenschaften bzw. Konsistenz in einem möglichst weiten Bereich einstellbar sind. Dabei sollen als hydrophobe Verbindungen siliconfreie Flüssigkeiten, Silicone und Mischungen davon einsetzbar sein. Bevorzugt soll die wasserunlösliche Verbindung bei Temperaturen > 40 °C ohne Lösungsmittel gut in die Formulierung einrührbar sein. Vorzugsweise sollen die Zusammensetzungen die Herstellung von kosmetischen, pharmazeutischen und technischen Mitteln ermöglichen, deren rheologische Eigenschaften sich in einem weiten Bereich von der flüssigen bis zur festen Form einstellen lassen.

Überraschenderweise wurde nun gefunden, dass diese Aufgabe durch ein Polyurethan gelöst wird, das
a) mindestens ein Diisocyanat,
b) mindestens ein Polytetrahydrofurane mit zwei aktiven Wasserstoffatomen pro Molekül und
c) mindestens eine Verbindung der allgemeinen Formel I

   R¹ - Z¹ (I)

   worin
   - R¹: für C₈- bis C₃₀-Alkyl, C₈- bis C₃₀-Alkenyl, Aryl-C₆-C₂₂-alkyl oder Aryl-C₆-C₂₂-alkenyl steht, wobei die Alkenylgruppen 1,2,3 oder 4 nicht benachbarte Doppelbindungen aufweisen, und
   - Z¹: für OH, SH oder NHR² steht, wobei R² für Wasserstoff, C₁- bis C₆-Alkyl oder C₅- bis C₈-Cycloalkyl steht,
   und zusätzlich wenigstens eine Komponente enthält, die ausgewählt ist unter
d) Polysiloxanen mit wenigstens zwei aktiven Wasserstoffatomen pro Molekül,
e) von a) bis d) verschiedenen Verbindungen mit einem Molekulargewicht im Bereich von 56 bis 600, die zwei aktive Wasserstoffatome pro Molekül enthalten
und Mischungen davon.

Im Rahmen der vorliegenden Erfindung umfasst der Ausdruck "Polyurethane" auch Polymere, die Harnstoffgruppen und/oder Thioharnstoffgruppen statt oder zusätzlich zu den Urethangruppen aufweisen.

Bevorzugt weisen die Polyurethane 2 bis 50, insbesondere 3 bis 45 Urethan- und/oder Harnstoffgruppen pro Molekül auf.

Bevorzugt sind Polyurethane, die ein Molekulargewicht im Bereich von etwa 500 bis 10 000, bevorzugt 700 bis 9 000, insbesondere 1 000 bis 8 000, aufweisen.

Vorzugsweise weisen die Polyurethane keine freien Isocyanatgruppen auf.

Vorzugsweise sind die Diisocyanate a) ausgewählt unter aliphatischen, cycloaliphatischen und/oder aromatischen Diisocyanaten, wie Tetramethylendiisocyanat, Hexamethylendiisocyanat, Methylendiphenyldiisocyanat, 2,4- und 2,6-Toluylendiisocyanat und deren Isomerengemische, o-, m- und p-Xylylendiisocyanat, 1,5-Naphthylendiisocyanat, 1,4-Cyclohexylendiisocyanat, Dicyclohexylmethandiisocyanat und Mischungen davon, insbesondere Isophorondiisocyanat, Hexamethylendiisocyanat und/oder Dicyclohexylmethandiisocyanat. Besonders bevorzugt wird Hexamethylendiisocyanat eingesetzt. Gewünschtenfalls können bis zu 3 Mol-% der genannten Verbindungen durch Triisocyanate ersetzt sein.

Bei der Komponente b) handelt es sich um Polytetrahydrofurane.

Geeignete Polytetrahydrofurane (Polytetramethylenglykole) b) können z. B. durch kationische Polymerisation von Tetrahydrofuran in Gegenwart von sauren Katalysatoren, wie z. B. Schwefelsäure oder Fluoroschwefelsäure, hergestellt werden. Derartige Herstellungsverfahren sind dem Fachmann bekannt. Das zahlenmittlere Molekulargewicht der Poly(THF)-Komponente b) liegt vorzugsweise in einem Bereich von etwa 250 bis 6000 g/mol, besonders bevorzugt 500 bis 5000 g/mol, insbesondere 650 bis 4000 g/mol.

Vorzugsweise umfasst die Komponente c) wenigstens eine Verbindung der allgemeinen Formel I, worin R¹ für einen geradkettigen oder verzweigten C₈- bis C₃₀-Alkyl- oder C₈- bis C₃₀-Alkenylrest steht, wobei der Alkenylrest 1, 2, 3 oder 4 nicht benachbarte Doppelbindungen aufweisen kann. Vorzugsweise handelt es sich um geradkettige und verzweigte C₁₂- bis C₂₈-, besonders bevorzugt um C₁₆- bis C₂₆-Alkyl- und Alkenylreste. Bevorzugt handelt es sich dabei um überwiegend lineare Alkyl- und/oder Alkenylreste, wie sie auch in natürlichen oder synthetischen Fettsäuren und Fettalkoholen sowie Oxoalkoholen vorkommen.

Bevorzugt steht der Rest R¹ in der Formel I für n-Octyl, Ethylhexyl, 1,1,3,3-Tetramethylbutyl, n-Nonyl, n-Decyl, n-Undecyl, n-Dodecyl, n-Tridecyl, Myristyl, Pentadecyl, Palmityl (=Cetyl), Heptadecyl, Octadecyl (= Stearyl), Nonadecyl, Arrachinyl, Behenyl, Lignocerenyl, Cerotinyl, Melissinyl etc. Besonders bevorzugte Reste R¹ sind Heptadecyl und Stearyl.

Vorzugsweise handelt es sich bei der Verbindung der allgemeinen Formel I um wenigstens einen primären Alkohol, Thioalkohol oder ein primäres Amin, worin R¹ für einen C₈- bis C₃₀-Alkyl- oder Alkenylrest steht. Die Verbindung der Formel I steht dann bevorzugt für 1-Octyl-, 1-Nonyl-, 1-Decyl-, 1-Undecyl-, 1-Undec-10-enyl-, 1-Tridecyl-, 1-Tetradecyl-, 1-Pentadecyl-, 1-Hexadecyl-, 1-Heptadecyl-, 1-Octadecyl-, 1-Octadeca-9,12-dienyl-, 1-Nonadecyl-, 1-Eicosyl-, 1-Eicos-9-enyl-, 1-Heneicosyl-, 1-Docosyl-Alkohol, Thiol und/oder Amin. Besonders bevorzugt steht die Verbindung der Formel I für Stearylalkohol und/oder Stearylamin.

Bei den Polysiloxanen d) handelt es sich vorzugsweise um eine Verbindung der Formel II 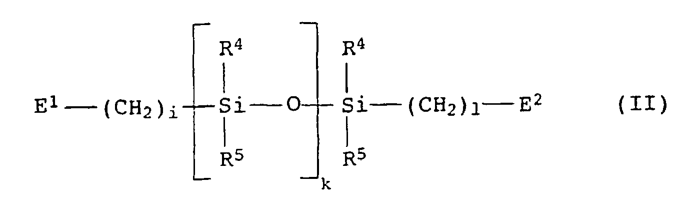 worin
- R⁴ und R⁵: unabhängig voneinander für C₁- bis C₄-Alkyl, Benzyl oder Phenyl stehen,
- E¹ und E²: unabhängig voneinander für OH oder NHR⁶ stehen, wobei R⁶ für Wasserstoff, C₁- bis C₆-Alkyl oder C₅- bis C₈-Cycloalkyl steht,
- i und l: unabhängig voneinander für 2 bis 8 stehen,
- k: für 3 bis 50 steht,
und Mischungen davon.

Geeignete Alkylreste sind z. B. Methyl, Ethyl, n-Propyl, Isopropyl, n-Butyl, t.-Butyl, n-Pentyl, n-Hexyl etc. Geeignete Cycloalkylreste sind z. B. Cyclopentyl, Cyclohexyl, Cycloheptyl, cyclooctyl etc.

Vorzugsweise stehen R⁴ und R⁵ beide für Methyl.

Diese Polysiloxane d) weisen vorzugsweise ein zahlenmittleres Molekulargewicht im Bereich von etwa 300 bis 5000, bevorzugt 400 bis 3000, auf.

Bevorzugt ist die Komponente e) ausgewählt unter Verbindungen der allgemeinen Formel III

X-R⁷-Y (III)

worin
- X und Y: unabhängig voneinander für OH oder NHR⁸ stehen, wobei R⁸ für Wasserstoff, C₈- bis C₃₀-Alkyl oder C₈- bis C₃₀-Alkenyl steht, wobei der Alkenylrest 1, 2, 3 oder 4 nicht benachbarte Doppelbindungen aufweisen kann, und
- R⁷: für eine zweiwertige verbrückende Gruppe mit 2 bis 25 Atomen in der Kette zwischen den flankierenden Bindungen steht.

Bevorzugt steht R⁷ für eine C₂- bis C₁₀-Alkylenbrücke die eine, zwei oder drei nicht benachbarte Doppelbindungen und/oder einen, zwei oder drei Substituenten, die ausgewählt sind unter Alkyl, Alkoxy, Hydroxy, Cycloalkyl und Aryl aufweisen kann, wobei die Alkylenbrücke zusätzlich durch ein, zwei oder drei nicht benachbarte, gegebenenfalls substituierte Heteroatome unterbrochen sein kann und/oder die Alkylenbrücke ein-, zwei- oder dreifach mit Aryl anelliert sein kann.

Bei der Komponente e) handelt es sich bevorzugt um Diole, Diamine, Aminoalkohole, und Mischungen davon. Das Molekulargewicht dieser Verbindungen liegt einem Bereich von 56 bis 500. Gewünschtenfalls können bis zu 3 Mol-% der genannten Verbindungen durch Triole oder Triamine ersetzt sein.

Bevorzugt werden als Komponente e) Diole eingesetzt. Brauchbare Diole sind z. B. Ethylenglykol, Propylenglykol, Butylenglykol, Neopentylglykol, Cyclohexandimethylol, Di-, Tri-, Tetra-, Penta- oder Hexaethylenglykol und Mischungen davon. Bevorzugt werden Hexaethylenglykol, Neopentylglykol und/oder Cyclohexandimethylol eingesetzt.

Bevorzugt werden als Komponente e) weiterhin Verbindungen der Formel X-(CH₂)₁₋₄-O-(P-C₆H₄)-CH₂-(P-C₆H₄)-O-(CH₂)₁₋₄-Y eingesetzt, worin X und Y unabhängig voneinander für OH oder NHR⁹ stehen, wobei R⁹ für Wasserstoff, C₁- bis C₈-Alkyl oder C₅- bis C₈-Cycloalkyl steht.

Besonders bevorzugt als Komponente e) ist 2,2- Bis(4-(hydroxyethyloxy)phenyl)methan, das unter dem Namen Dianol® 22 (Fa. Akzo Nobel) kommerziell erhältlich ist.

Geeignete Aminoalkohole e) sind z. B. 2-Aminoethanol, 2-(N-Methylamino)ethanol, 3-Aminopropanol, 4-Aminobutanol, 1-Ethylaminobutan-2-ol, 2-Amino-2-methyl-1-propanol, 4-Methyl-4-aminopentan-2-ol etc.

Geeignete Diamine e) sind z. B. Ethylendiamin, Propylendiamin, 1,4-Diaminobutan, 1,5-Diaminopentan und 1,6-Diaminohexan.

Bevorzugte Diamine e) sind Diamine der Formel R^{a}-NH-(CH₂)₂₋₃-NH₂, wobei R^{a} für C₁₀- bis C₃₀-Alkyl oder C₁₀- bis C₃₀-Alkenyl steht, wobei der Alkenylrest 1, 2 oder 3 nicht benachbarte Doppelbindungen aufweisen kann. Das Molekulargewicht dieser Diamine e) liegt vorzugsweise in einem Bereich von etwa 160 bis 400.

Weiterhin geeignete Diamine e) sind z. B. Hexamethylendiamin, Piperazin, 1,2-Diaminocyclohexan, 1,3-Diaminocyclohexan, 1,4-Diaminocyclohexan, Neopentandiamin, 4,4'-Diaminodicyclohexylmethan etc.

Die Herstellung der erfindungsgemäßen Polyurethane erfolgt z. B. durch Umsetzung wenigstens eines Diisocyanats a) mit den gegenüber Isocyanatgruppen reaktiven Gruppen der Komponenten b), c) und d) und/oder e). Vorzugsweise erfolgt die Umsetzung bei erhöhten Temperaturen im Bereich von etwa 60 bis 140°C, insbesondere etwa 70 bis 100°C. Die Reaktion kann ohne Lösungsmittel in der Schmelze oder in einem geeigneten Lösungsmittel oder Lösungsmittelgemisch erfolgen. Geeignete Lösungsmittel sind z. B. aprotisch polare Lösungsmittel, wie Tetrahydrofuran, Essigsäureethylester, N-Methylpyrrolidon, Dimethylformamid und bevorzugt Ketone, wie Aceton und Methylethylketon. Werden die erfindungsgemäßen Polyurethane als Komponente einer Zusammensetzung eingesetzt, die wenigstens eine hydrophobe flüssige Verbindung (Öl- oder Fettkomponente) aufweist, so erfolgt die Herstellung der Polyurethane vorzugsweise in dieser Ölkomponente als Lösungsmittel. Bevorzugt erfolgt die Reaktion unter einer Inertgasatmosphäre, wie z. B. unter Stickstoff. Die Komponenten werden bevorzugt in solchen Mengen eingesetzt, das das Verhältnis von NCO-Äquivalent der Verbindungen der Komponente a) zu Äquivalent aktives Wasserstoffatom der Komponenten b), c) und gegebenenfalls d) und/oder e) in einem Bereich von etwa 0,9:1 bis 1,1:1 liegt.

Gegenstand der Erfindung ist die Verwendung wenigstens eines Polyurethans, wie zuvor definiert, als Komponente einer homogenphasigen oder heterogenphasigen Zusammensetzung, die 20 bis 99.9 Gew.-% wenigstens einer wasserlöslichen, bei 20°C flüssigen Verbindung enthält, zur Modifizierung der rheologischen Eigenschaften dieser Zusammensetzung.

Ein weiterer Gegenstand der Erfindung ist ein Verfahren zur Modifizierung der rheologischen Eigenschaften einer homogenphasigen oder heterogenphasigen Zusammensetzung, die 20 bis 99.9 Gew.-% wenigstens einer wasserlöslichen, bei 20°C flüssigen Verbindung enthält, wobei man die Zusammensetzung mit wenigstens einem Polyurethan wie zuvor definiert, mischt und gegebenenfalls erwärmt.

"Modifizierung rheologischer Eigenschaften" wird im Rahmen der vorliegenden Erfindung weit verstanden. Die erfindungsgemäßen Polyurethane eignen sich im Allgemeinen zur Verdickung der Konsistenz von hydrophoben Verbindungen in einem weiten Bereich. Je nach Grundkonsistenz der hydrophoben flüssigen Verbindung(en) können in Abhängigkeit von der Einsatzmenge des Polyurethans in der Regel Fließeigenschaften von dünflüssig bis hin zu fest (nicht mehr fließend) erzielt werden. Unter "Modifizierung rheologischer Eigenschaften" wird daher unter anderem die Erhöhung der Viskosität von Flüssigkeiten, die Verbesserung der Thixotropie-Eigenschaften von Gelen, die Verfestigung von Gelen und Wachsen etc. verstanden.

"Homogenphasige Zusammensetzung" weisen unabhängig von der Anzahl ihrer Bestandteile nur eine einzige Phase auf. "Heterogenphasige Zusammensetzungen" sind disperse Systeme von zwei oder mehreren miteinander nicht mischbaren Komponenten. Dazu zählen vorzugsweise Emulsionen, wie z. B. O/W und W/O-Formulierungen, die wenigstens eine der zuvor beschriebenen Öl- bzw. Fettkomponenten und Wasser als nicht mischbare Phasen aufweisen. Dabei werden die erfindungsgemäßen Polyurethane im Allgemeinen in der Ölphase eingesetzt.

Geeignete wasserlösliche, bei 20°C flüssige Verbindungen, deren rheologische Eigenschaften durch die erfindungsgemäßen Polyurethane modifiziert werden können, werden im Folgenden als Komponente B) genannt.

Ein weiterer Gegenstand der Erfindung ist eine Zusammensetzung, enthaltend
A) wenigstens ein Polyurethan, wie zuvor definiert,
B) 20 bis 99.9 Gew.-% wenigstens einer wasserunlöslicher, bei 20°C flüssigen Verbindung.

Vorzugsweise enthalten die erfindungsgemäßen Zusammensetzungen wenigstens ein Polyurethan A), das
- 10 bis 30 Gew.-%, bevorzugt 12 bis 25 Gew.-%, wenigstens eines Diisocyanats a),
- 30 bis 80 Gew.-%, bevorzugt 35 bis 70 Gew.-%, wenigstens eines Polytetrahydrofurane b),
- 9 bis 30 Gew.-%, bevorzugt 7 bis 25 Gew.-%, wenigstens einer Komponente c),
- 0 bis 40 Gew.-%, bevorzugt 0,01 bis 35 Gew.-%, insbesondere 0,1 bis 25 Gew.-%, wenigstens eines Polysiloxans d), und
- 0 bis 20 Gew.-%, bevorzugt 1 bis 18 Gew.-%, insbesondere 3 bis 15 Gew.-%, wenigstens einer Komponente e)
einpolymerisiert enthält.

Nach einer bevorzugten Ausführungsform enthalten die erfindungsgemäßen Zusammensetzungen Siloxangruppen-haltige Polyurethane. Nach einer weiteren bevorzugten Ausführungsform enthalten die erfindungsgemäßen Zusammensetzungen wenigstens ein siloxanfreies Polyurethan. Für Siloxangruppen-haltige Polyurethane liegt das Gewichtsmengenverhältnis von Polyether b) zu Polysiloxankompente d) vorzugsweise in einem Bereich von etwa 99,9:0,1 bis 50:50, bevorzugt 99:1 bis 60:40, insbesondere 95:5 bis 80:20.

Die erfindungsgemäß eingesetzten Siloxangruppen-freien Polyurethane enthalten als Komponente b) wenigstens ein Polytetrahydrofuran.

Die erfindungsgemäßen Zusammensetzungen weisen 20 bis 99.9 Gew.-% wenigstens einer im Wesentlichen wasserunlöslichen (hydrophoben) Flüssigkeit B) (Öl-bzw. Fettkomponente) auf. Unter wasserunlöslich wird eine Wasserlöslichkeit von in der Regel höchstens 1 g/l bei 20°C verstanden.

Die erfindungsgemäßen Zusammensetzungen weisen bevorzugt wenigstens eine Komponente B) auf, die ausgewählt ist unter
- Ölen, bevorzugt Mineralölen, vollsynthetischen Ölen, Ölen pflanzlichen und tierischen Ursprungs und etherischen Ölen,
- Fetten,
- gesättigten acyclischen und cyclischen Kohlenwasserstoffen,
- Estern von Monocarbonsäuren mit ein-, zwei- oder dreiwertigen Alkoholen,
- Siliconölen,
und Mischungen davon.

Die erfindungsgemäßen Zusammensetzungen weisen z. B. eine Öl- bzw. Fettkomponente B) auf, die ausgewählt ist unter: Kohlenwasserstoffen geringer Polarität, wie Mineralölen; linearen gesättigten Kohlenwasserstoffen, wie Tetradecan, Hexadecan, Octadecan etc.; cyclischen Kohlenwasserstoffen, wie Decahydronaphthalin; verzweigten Kohlenwasserstoffen; Estern, bevorzugt Estern von Fettsäuren, wie z. B. die Ester von C₁- bis C₂₄-Monoalkoholen mit C₁- bis C₂₂-Monocarbonsäuren, wie Isopropylisostearat, n-Propylmyristat, iso-Propylmyristat, n-Propylpalmitat, iso-Propylpalmitat, Hexacosanylpalmitat, Octacosanylpalmitat, Triacontanylpalmitat, Dotriacontanylpalmitat, Tetratriacontanylpalmitat, Hexancosanylstearat, Octacosanylstearat, Triacontanylstearat, Dotriacontanylstearat, Tetratriacontanylstearat; Salicylaten, wie C₁- bis C₁₀-Salicylaten, z. B. Octylsalicylat; Benzoatestern, wie C₁₀- bis C₁₅-Alkylbenzoaten, Benzylbenzoat; anderen kosmetischen Estern, wie Fettsäuretriglyceriden, Propylenglykolmonolaurat, Polyethylenglykolmonolaurat, Castoröl, C₁₀- bis C₁₅-Alkyllactaten, etc.

Geeignete Siliconöle B) sind z. B. lineare Polydimethylsiloxane, Poly(methylphenylsiloxane), cyclische Siloxane und Mischungen davon. Das zahlenmittlere Molekulargewicht der Polydimethylsiloxane und Poly(methylphenylsiloxane) liegt vorzugsweise in einem Bereich von etwa 1000 bis 150000 g/mol. Bevorzugte cyclische Siloxane weisen 4- bis 8-gliedrige Ringe auf. Geeignete cyclische Siloxane sind z. B. unter der Bezeichnung Cyclomethicon kommerziell erhältlich.

Die erfindungsgemäßen ölhaltigen bzw. fetthaltigen Zusammensetzungen enthalten diese Komponente B) in einer Menge von etwa 20 bis 99,9 Gew.-%, bevorzugt 20 bis 90 Gew.-%, insbesondere 20 bis 80 Gew.-%, bezogen auf das Gesamtgewicht der Zusammensetzung.

Die erfindungsgemäßen Zusammensetzungen sowie öl- bzw. fetthaltige Produkte auf deren Basis lassen sich im Allgemeinen bezüglich ihrer rheologischen Eigenschaften bzw. ihrer Konsistenz in einem weiten Bereich einstellen. Die erfindungsgemäßen Zusammensetzungen eignen sich somit vorzugsweise zur Formulierung von öl- bzw. fetthaltigen kosmetischen, pharmazeutischen und technischen Produkten. Je nach Grundkonsistenz der Komponente B) sowie gegebenenfalls zur Formulierung von Produkten zusätzlich eingesetzter Komponenten können die Eigenschaften im Allgemeinen in Abhängigkeit von der Einsatzmenge des Polyurethans A) von einer dünnflüssigen bis zu einer festen Konsistenz variiert werden. Vorzugsweise sind dabei die Lösungen der Polyurethane A) in der Komponente B) im Allgemeinen klar. Vorteilhafterweise können somit Formulierungen, insbesondere kosmetische Formulierungen, ohne Beeinträchtigung durch die Eigenfarbe der erfindungsgemäßen Zusammensetzung angefärbt werden.

Nach einer bevorzugten Ausführungsform enthalten die erfindungsgemäßen Zusammensetzungen wenigstens ein Polyurethan A), das wenigstens ein Polysiloxan d) eingebaut enthält, und eine Komponente B), die wenigstens ein Siliconöl umfasst.

Nach einer weiteren bevorzugten Ausführungsform ist entweder das Polyurethan A) oder die Komponente B) siliconfrei. Insbesondere sind sowohl das Polyurethan A) als auch die Komponente B) siliconfrei. Uberraschenderweise weisen die erfindungsgemäßen Zusammensetzungen, die siliconfreie Komponenten A) und/oder B) umfassen, dennoch gute anwendungstechnische Eigenschaften auf. So sind z. B. die rheologischen Eigenschaften dieser Zusammensetzungen in der Regel in vorteilhafter Weise einstellbar.

Ein weiterer Gegenstand der Erfindung ist die Verwendung von Zusammensetzungen, wie zuvor definiert als oder in kosmetische(n) und pharmazeutische(n) Zubereitung(en), als oder in Beschichtungs- und Behandlungsmitteln für nichtsaugende Oberflächen, bevorzugt Metalle, Kunststoffe, Textilsynthesefasern und Glas, und für saugfähige Oberflächen, bevorzugt Holz, Papier, Baumwolle und Leder.

Ein weiterer Gegenstand der Erfindung ist die Verwendung von Zusammensetzungen, wie zuvor definiert, zur Herstellung von Kerzen, Brenn- und Kraftstoffen, industriellen Fetten, Rostschutzmitteln und Tintenstrahldruckerpatronen.

Ein weiterer Gegenstand der Erfindung ist die Verwendung von Polyurethanen A), wie zuvor beschrieben, als Komponente pharmazeutischer, kosmetischer und technischer Zubereitungen bevorzugt in kosmetischen Zubereitungen zur Behandlung der Haut, sowie zur Modifizierung der rheologischen Eigenschaften von Zusammensetzungen auf Basis von Verbindungen geringer Polarität.

Ein weiterer Gegenstand der Erfindung ist ein kosmetisches oder pharmazeutisches Mittel, enthaltend
- wenigstens ein Polyurethan, wie zuvor definiert,
- wenigstens eine wasserunlösliche, bei 20°C flüssige Verbindung,
- 20 bis 99.9 Gew.-% wenigstens einer kosmetisch oder pharmazeutisch aktiven Substanz, und
- gegebenenfalls wenigstens einen Zusatzstoff.

Nach einer bevorzugten Ausführungsform liegen die erfindungsgemäßen kosmetischen Mittel in Form eines ölhaltigen oder fetthaltigen kosmetischen Präparates vor. Dazu zählen z. B. Cremes, Mascara, Augen-Make-up, Gesichts-Make-up, kosmetische Öle, Babyöl, Badeöl, Make-up-Entferner, Hautfeuchthaltemittel, Sonnenschutzmittel, Lippenpflegemittel, wasserfreie Handwaschmittel, medizinische Salben etc.

Die erfindungsgemäßen ölhaltigen bzw. fetthaltigen kosmetischen Mittel enthalten die Komponente B) in einer Menge von 20 bis 99,9 Gew.-%, bevorzugt 20 bis 90 Gew.-%, insbesondere 20 bis 80 Gew.-%, bezogen auf das Gesamtgewicht des Mittels.

Die erfindungsgemäßen ölhaltigen bzw. fetthaltigen kosmetischen Mittel enthalten die zuvor beschriebenen Polyurethane A) im Allgemeinen in einer Menge von etwa 0,1 bis 50 Gew.-%, bevorzugt 0,2 bis 30 Gew.-%, insbesondere 0,5 bis 10 Gew.-%, bezogen auf die Gesamtmenge des Mittels.

Weiterhin können die ölhaltigen bzw. fetthaltigen kosmetischen Mittel Hilfs- und/oder Zusatzstoffe, wie Emulgatoren, Überfettungsmittel, Stabilisatoren, Wachse, Konsistenzgeber, Verdickungsmittel, Siliconverbindungen, biogene Wirkstoffe, Filmbildner, Konservierungsmittel, Hydrotrope, Solubilisatoren, UV-Adsorber, Farb- und Duftstoffe etc. enthalten.

Die erfindungsgemäßen öl- bzw. fetthaltigen kosmetischen Produkte auf Basis der zuvor beschriebenen Polyurethane lassen sich im Allgemeinen bezüglich ihrer rheologischen Eigenschaften bzw. ihrer Konsistenz in einem weiten Bereich einstellen. Je nach Grundkonsistenz des kosmetischen Mittels können die Eigenschaften im Allgemeinen in Abhängigkeit von der Einsatzmenge des Oligomers von einer dünnflüssigen bis zu einer festen Konsistenz variiert werden. Vorteilhafterweise lassen sich somit hautkosmetische Produkte formulieren, die einen hohen Anteil an dünnflüssigen öl- bzw. fetthaltigen Komponenten aufweisen.

Vorteilhafterweise eignen sich die beschriebenen Polyurethane und die erfindungsgemäßen Zusammensetzungen auf deren Basis zur Formulierung von Gelen. Unter "Gel" versteht man im Allgemeinen eine Formulierung, die eine höhere viskosität als eine Flüssigkeit aufweist und die selbsttragend ist, d. h. eine ihr verliehene Form ohne formstabilisierende Umhüllung behält. Zur Formulierung von Gelen eignen sich im Allgemeinen alle zuvor genannten Ölkomponenten, die bei Umgebungstemperatur flüssig sind. Vorteilhafterweise sind Gele auf Basis der zuvor beschriebenen Polyurethane im Allgemeinen transparent. Sie können mit üblichen Zusatzstoffen zu erfindungsgemäßen kosmetischen Mitteln, wie z. B. Lippenpflegemitteln, Desodorantien, Antiperspirantien, Make-ups, etc., formuliert werden. Vorteilhafterweise können die beschriebenen Polyurethane und die erfindungsgemäßen Zusammensetzungen auf deren Basis auch zur Herstellung nicht-kosmetischer Produkte auf Gelbasis eingesetzt werden. Dazu zählen z. B. Autowachse und -polituren, Kerzen, Möbelpolituren, Metallreiniger, Haushaltsreiniger, etc.

Vorteilhafterweise eignen sich die beschriebenen Polyurethane und die erfindungsgemäßen Zusammensetzungen auf deren Basis auch zur Herstellung üblicher O/W- und W/O-Formulierungen, wie z. B. Cremes, wobei die polyurethane im Allgemeinen in der Ölphase eingesetzt werden.

Die beschriebenen Polyurethane eignen sich insbesondere als Verdickungsmittel für Flüssigkeiten niederer Polarität, vorzugsweise Öle. Bevorzugt werden Polyurethane als Verdicker für Öle eingesetzt, die einen auf das Gesamtgewicht bezogenen Anteil von Urethan- und/oder Harnstoffgruppen von höchstens 5 Gew.-% aufweisen. Diese Komponenten weisen vorzugsweise eine hohe Verträglichkeit mit nicht-siliconhaltigen Ölen auf. Sie sind im Allgemeinen in Siliconölen, nicht-siliconhaltigen Ölen oder Mischungen davon löslich. Vorteilhafterweise sind die dabei erhaltenen Lösungen im Allgemeinen klar. Vorteilhafterweise lassen sich klare kosmetische Formulieren z. B. leichter anfärben als bereits gefärbte. Die beschriebenen Polyurethane und die erfindungsgemäßen Zusammensetzungen auf deren Basis eignen sich vorzugsweise für die Verwendung in Personal-Care-Produkte, wie beispielsweise kosmetischen Mitteln, z. B. Augen-Make-up, Gesichts-Make-up, Babyöl, Badeöl, Make-up-Entferner, Hautfeuchthaltemitteln, Sonnenschutzmitteln, Lippenpflegemitteln, wasserfreien Handwaschmitteln, medizinischen Salben, Parfums und Suppositorien. Sie eignen sich weiterhin vorteilhafterweise zur Formulierung haarkosmetischer Produkte, wie Haarsprays, Schaumfestiger, Haarmousse, Haargel und Shampoos. Sie eignen sich weiterhin bevorzugt für eine Verwendung in der dekorativen Kosmetik, insbesondere in Mascara und Lidschatten. Ferner können die zuvor beschriebenen Polyurethane und deren Reaktionsprodukte vorteilhaft in Haushaltsprodukten, wie Autowachsen und -polituren, Kerzen, Möbelpolituren, Metallreinigungsmitteln und Metallpolituren, Haushaltsreinigern, Farbentfernern und Trägermaterialien für Insektizide eingesetzt werden. Sie eignen sich weiterhin für die Anwendung in technischen bzw. industriellen Produkte, wie beispielsweise in Kraftstoffen, Fetten, Lötfetten, Rostschutzmitteln und Tintenstrahldruckerpatronen.

Die Erfindung wird anhand der folgenden, nicht einschränkenden Beispiele näher erläutert.

### Beispiele

### I. Polyurethanherstellung

### Polyurethan 1:

In einem Vierhalskolben, der mit Rührer, Tropftrichter, Thermometer, Rückflusskühler und einer Vorrichtung für das Arbeiten unter stickstoff ausgestattet war, wurden 58,38 g Polytetrahydrofuran (Mₙ = 1 000 g/mol), 6,19 g Diethylenglykol, 15,79 g Stearylalkohol und 0,1 g Tetrabutylorthotitanat vorgelegt und die Mischung unter Rühren auf etwa 70 °C erwärmt und homogenisiert. Anschließend wurden unter Rühren 19,64 g Hexamethylendiisocyanat zugetropft, wobei die Reaktionstemperatur anstieg. Das Reaktionsgemisch wurde weiter bei 80 °C gerührt, bis der NCO-Gehalt bei weniger als 0.01 Mol-% des Ausgangsgehalts lag (ca. 4 bis 5 h).

### Polyurethan 2:

Analog zu Polyurethan 1 wurde ein Polyurethan aus 61,26 g Polytetrahydrofuran (Mₙ = 1 000 g/mol), 6,03 g 1,6-Hexandiol, 13,81 g Stearylalkohol und 18,89 g Hexamethylendiisocyanat hergestellt.

### Polyurethan 3:

Analog der Herstellungsvorschrift für das Polyurethan 1 wurde ein Polyurethan aus 61,26 g Polytetrahydrofuran (Mₙ = 1 000 g/mol), 6,03 g 1,6-Hexandiol, 13,81 g Stearylalkohol und 18,89 g Hexamethylendiisocyanat bei einer Temperatur von etwa 80°C in 30 ml Aceton als Lösungsmittel hergestellt.

### Polyurethan 4:

Analog der Herstellungsvorschrift für Polyurethan 3 wurde ein Polyurethan aus 42,67 g Polytetrahydrofuran (Mₙ = 1 000 g/mol), 12,72 g Duomeen® T der Firma Ceca (C₁₂- bis C₂₄-Alkylfettdiamin), 23,08 g Stearylalkohol und 21,53 g Hexamethylendiisocyanat hergestellt.

### Polyurethan 5:

Analog der Herstellungsvorschrift für Polyurethan 3 wurde in Methylethylketon als Lösungsmittel ein Polyurethan aus 42,34 g Polytetrahydrofuran (Mₙ = 1 000 g/mol), 13,38 g 2,2-Bis(4-(hydroxyethyloxy)phenyl)methan (Dianol® 22 der Firma Akzo Nobel), 22,91 g Stearylalkohol und 21,37 g Hexamethylendiisocyanat hergestellt.

Zur Herstellung von Formulierungen der zuvor genannten Polyurethane werden diese mit der Ölkomponente gemäß Tabelle 1 gemischt und auf eine Temperatur von 50 bis 180 °C erhitzt, bis das Polyurethan vollständig in Lösung gegangen ist. Nach dem Abkühlen erhält man eine Mischung mit modifizierten rheologischen Eigenschaften. Die Eigenschaften der Mischungen sind ebenfalls in Tabelle 1 wiedergegeben.

Anwendungstechnische Eigenschaften

**Tabelle 1:**

| Polyurethan aus Bsp. | Öl | Polyurethankonzentration [Gew.-%] | Aussehen der erkalteten Zusammensetzung |
|---|---|---|---|
| 1 | Finsolv® TN | 10 | fest, leicht trüb |
| 2 | Finsolv® TN | 10 | fest, opaque |
| 3 | Paraffinöl | 10 | fest, opaque bis leicht trüb |
| 3 | Finsolv® TN | 10 | fest, opaque |
| 3 | Finsolv® TN | 20 | fest, opaque |
| 3 | Finsolv® TN/ Siliconöl 80/20 | 20 | fest, opaque bis leicht trüb |
| 4 | Finsolv® TN | 10 | dickflüssig, leicht trüb |
| 5 | Finsolv® TN | 10 | dickflüssig, leicht trüb |

| | | | |
|---|---|---|---|
| Finsolv® TN = C₁₂- bis C₁₅-Alkylbenzoat | | | |

## Patentansprüche

1. Verwendung wenigstens eines Polyurethans, das
a) mindestens ein Diisocyanat,
b) mindestens ein Polytetrahydrofuran mit zwei aktiven Wasserstoffatomen pro Molekül und
c) mindestens eine Verbindung der allgemeinen Formel I
R¹ - Z¹ (I)
worin
R¹ für C₈- bis C₃₀-Alkyl, C₈- bis C₃₀-Alkenyl, Aryl-C₆-C₂₂-alkyl oder Aryl-C₆-C₂₂-alkenyl steht, wobei die Alkenylgruppen 1,2,3 oder 4 nicht benachbarte Doppelbindungen aufweisen, und
Z¹ für OH, SH oder NHR² steht, wobei R² für Wasserstoff, C₁- bis C₆-Alkyl oder C₅- bis C₈-Cycloalkyl steht
und zusätzlich wenigstens eine Komponente, die ausgewählt ist unter
d) Polysiloxanen mit wenigstens zwei aktiven Wasserstoffatomen pro Molekül,
e) von a) bis d) verschiedenen Verbindungen mit einem Molekulargewicht im Bereich von 56 bis 600, die zwei aktive Wasserstoffatome pro Molekül enthalten und Mischungen davon,
eingebaut enthält,
als Komponente einer homogenphasigen oder heterogenphasigen Zusammensetzung, die bezogen auf das Gesamtgewicht der Zusammensetzung 20 bis 99,9 Gew.-% wenigstens einer wasserunlöslichen, bei 20°C flüssigen Verbindung enthält, zur Modifizierung der rheologischen Eigenschaften dieser Zusammensetzung.

2. Verwendung nach Anspruch 1, wobei es sich bei der Komponente a) um Hexamethylendiisocyanat handelt.

3. Verfahren zur Modifizierung der rheologischen Eigenschaften einer homogenphasigen oder heterogenphasigen Zusammensetzung, die bezogen auf das Gesamtgewicht der Zusammensetzung 20 bis 99,9 Gew.-% wenigstens einer wasserunlöslichen, bei 20°C flüssigen Verbindung enthält, wobei man die Zusammensetzung mit wenigstens einem Polyurethan, wie in einem der Ansprüche 1 oder 2 definiert, mischt und gegebenenfalls erwärmt.

4. Zusammensetzung, enthaltend
A) wenigstens ein Polyurethan, das besteht aus den Komponenten a), b), c) und wenigstens einer Komponente, die ausgewählt ist unter d) und e) wie in den Ansprüchen 1 oder 2 definiert, und
B) 20 bis 99,9 Gew.-%, bezogen auf das Gesamtgewicht der Zusammensetzung wenigstens einer wasserunlöslichen, bei 20°C flüssigen Verbindung.

5. Zusammensetzung nach Anspruch 4, wobei die Komponente B) ausgewählt ist unter
- Ölen, bevorzugt Mineralölen, vollsynthetischen Ölen, Ölen pflanzlichen und tierischen Ursprungs und etherischen Ölen,
- Fetten,
- gesättigten acyclischen und cyclischen Kohlenwasserstoffen,
- Estern von Monocarbonsäuren mit ein-, zwei- oder dreiwertigen Alkoholen,
- Siliconölen,
und Mischungen davon.

6. Verwendung von Zusammensetzungen, wie in einem der Ansprüche 4 oder 5 definiert, als oder in kosmetische(n) und pharmazeutische(n) Zubereitung(en), als oder in Beschichtungs- und Behandlungsmittel(n) für nichtsaugende Oberflächen, bevorzugt Metalle, Kunststoffe, Textilsynthesefasern und Glas, und für saugfähige Oberflächen, bevorzugt Holz, Papier, Baumwolle und Leder.

7. Verwendung von Zusammensetzungen, wie in einem der Ansprüche 4 oder 5 definiert, zur Herstellung von Kerzen, Brenn- und Kraftstoffen, industriellen Fetten, Rostschutzmitteln und Tintenstrahldruckerpatronen.

8. Kosmetisches oder pharmazeutisches Mittel, enthaltend
- wenigstens ein Polyurethan, wie in einem der Ansprüche 1 oder 2 definiert,
- 20 bis 99,9 Gew.-%, bezogen auf das Gesamtgewicht des Mittels wenigstens einer wasserunlöslichen, bei 20°C flüssigen Verbindung,
- wenigstens eine kosmetisch oder pharmazeutisch aktive Substanz,
- gegebenenfalls wenigstens einen Zusatzstoff.

## Claims

1. The use of at least one polyurethane which comprises, as incorporated units,
a) at least one diisocyanate,
b) at least one polytetrahydrofuran having two active hydrogen atoms per molecule and
c) at least one compound of the formula I
R¹ - Z¹ (I)
where
R¹ is C₈- to C₃₀-alkyl, C₈- to C₃₀-alkenyl, aryl-C₆-C₂₂-alkyl or aryl-C₆-C₂₂-alkenyl, the alkenyl groups having 1, 2, 3 or 4 nonneighboring double bonds, and
Z¹ is OH, SH or NHR², where R² is hydrogen, C₁- to C₆-alkyl or C₅- to C₈-cycloalkyl
and additionally at least one component which is selected from
d) polysiloxanes having at least two active hydrogen atoms per molecule,
e) compounds which differ from a) to d), have a molecular weight of from 56 to 600 and comprise two active hydrogen atoms per molecule and mixtures thereof,
as a component of a homogeneous or heterogeneous composition which, based on the total weight of the composition, comprises from 20 to 99.9% by weight of at least one water-insoluble compound liquid at 20°C for modifying the rheological properties of this composition.

2. The use according to claim 1, where component a) is hexamethylene diisocyanate.

3. A process for modifying the rheological properties of a homogeneous or heterogeneous composition which, based on the total weight of the composition, comprises from 20 to 99.9% by weight of at least one water-insoluble compound liquid at 20°C, the composition being mixed with at least one polyurethane, as defined in either of claims 1 and 2, and, if required, heated.

4. A composition comprising
A) at least one polyurethane consisting of components a), b), c) and at least one component selected from d) and e), as defined in claims 1 and 2, and
B) from 20 to 99.9% by weight, based on the total weight of the composition, of at least one water-insoluble compound liquid at 20°C.

5. The composition according to claim 4, the component B) being selected from
- oils, preferably mineral oils, fully synthetic oils, oils of vegetable and animal origin and essential oils,
- fats,
- saturated acyclic and cyclic hydrocarbons,
- esters of monocarboxylic acids with monohydric, dihydric or trihydric alcohols,
- silicone oils
and mixtures thereof.

6. The use of a composition, as defined in either of claims 4 and 5, as or in cosmetic and pharmaceutical formulation(s), as or in coating and treatment composition(s) for nonabsorptive surfaces, preferably metals, plastics, textile man-made fibers and glass, and for absorptive surfaces, preferably wood, paper, cotton and leather.

7. The use of a composition, as defined in either of claims 4 and 5, for the preparation of candles, combustion and power fuels, industrial greases, antirust compositions and ink jet printer cartridges.

8. A cosmetic or pharmaceutical composition comprising
- at least one polyurethane, as defined in either of claims 1 and 2,
- from 20 to 99.9% be weight, based on the total weight of the composition, of at least one water-insoluble compound liquid at 20°C,
- at least one cosmetically or pharmaceutically active substance and
- if appropriate, at least one additive.

## Revendications

1. Utilisation d'au moins un polyuréthanne, qui contient, de manière intégrée,
a) au moins un diisocyanate,
b) au moins un polytétrahydrofuranne comportant deux atomes d'hydrogène actifs par molécule et
c) au moins un composé de formule générale I :
R¹ - Z¹ (I)
dans laquelle :
R¹ est un groupement alkyle en C₈-C₃₀, alcényle en C₈-C₃₀, arylalkyle (C₆-C₂₂) ou arylalcényle (C₆-C₂₂), les groupements alcényle présentant 1, 2, 3 ou 4 doubles liaisons non adjacentes, et
Z¹ représente OH, SH ou NHR², où R² est de l'hydrogène, un alkyle en C₁-C₆ ou un cycloalkyle en C₅₋C₈, et,
en plus, au moins un composant choisi parmi :
d) des polysiloxanes comportant au moins deux atomes d'hydrogène actifs par molécule,
e) des composés différents de a) à d) avec un poids moléculaire dans la plage de 56 à 600, qui comportent deux atomes d'hydrogène actifs par molécule et leurs mélanges,
comme composant d'une composition à phase homogène ou à phase hétérogène, qui contient, par rapport au poids total de la composition, 20 à 99,9% en poids d'au moins un composé insoluble dans l'eau liquide à 20°C pour modifier les propriétés rhéologiques de cette composition.

2. Utilisation selon la revendication 1, dans laquelle, en ce qui concerne le composant a), il s'agit du diisocyanate d'hexaméthylène.

3. Procédé pour modifier les propriétés rhéologiques d'une composition à phase homogène ou à phase hétérogène qui contient, par rapport au poids total de la composition, 20 à 99,9% en poids d'au moins un composé insoluble dans l'eau liquide à 20°C, dans lequel on mélange et chauffe éventuellement la composition avec au moins un polyuréthanne, comme défini dans l'une des revendications 1 ou 2.

4. Composition contenant :
A) au moins un polyuréthanne, qui est constitué des composants a), b), c) et d'au moins un composant choisi parmi d) et e), comme défini dans l'une des revendications 1 ou 2, et
B) 20 à 99,9% en poids, par rapport au poids total de la composition, d'au moins un composé insoluble dans l'eau liquide à 20°C.

5. Composition selon la revendication 4, dans laquelle le composant B) est choisi parmi :
- des huiles, de préférence des huiles minérales, des huiles entièrement synthétiques, des huiles d'origine végétale et animale et des huiles essentielles,
- des graisses,
- des hydrocarbures saturés acycliques et cycliques,
- des esters d'acides monocarboxyliques avec des alcools monovalents, bivalents ou trivalents,
- des huiles de silicones, et leurs mélanges.

6. Utilisation de compositions, comme défini dans l'une des revendications 4 ou 5, sous la forme ou au sein d'une ou plusieurs préparations cosmétiques et pharmaceutiques, sous la forme ou au sein d'un ou plusieurs agents de revêtement et de traitement pour des surfaces non absorbantes, de préférence des métaux, des matériaux plastiques, des fibres textiles synthétiques et du verre, et pour des surfaces absorbantes, de préférence le bois, le papier, le coton et le cuir.

7. Utilisation de compositions, comme défini dans l'une des revendications 4 ou 5, pour la fabrication de bougies, de combustibles et de carburants, de graisses industrielles, d'agents antirouille et de cartouches d'impression pour jet d'encre.

8. Agent cosmétique ou pharmaceutique contenant :
- au moins un polyuréthanne, comme défini dans l'une des revendications 1 ou 2,
- 20 à 99,9% en poids, par rapport au poids total de l'agent, d'au moins un composé insoluble dans l'eau liquide à 20°C,
- au moins une substance active au plan cosmétique ou pharmaceutique, et
- éventuellement au moins un additif.
